# EUROPEAN PATENT SPECIFICATION

(11) **EP 3 289 869 B1**
(45) Date of publication and mention of the grant of the patent: **14.10.2020**
(21) Application number: 16786561.7
(22) Date of filing: 28.04.2016
(51) Int. Cl.: A01K 67/027, C12N 15/09, C12Q 1/68, G01N 33/15, G01N 33/50

(54) **TRANSGENIC NON-HUMAN ANIMAL EXPRESSING HUMAN-SPECIFIC MOLECULES AND HUMAN FC GAMMA RECEPTOR FAMILY**
TRANSGENES NICHTHUMANES TIER ZUR EXPRESSION VON HUMANSPEZIFISCHEN MOLEKÜLEN UND HUMANER FC GAMMA -REZEPTOR-FAMILIE
ANIMAL NON-HUMAIN TRANSGÉNIQUE EXPRIMANT DES MOLÉCULES SPÉCIFIQUES À UN ÊTRE HUMAIN ET FAMILLE DE RÉCEPTEURS FC GAMMA HUMAINS

(30) Priority: 30.04.2015 JP 2015093445
(43) Date of publication of application: 07.03.2018
(73) Proprietor: Institute Of Immunology Co., Ltd., Tokyo 112-0004 (JP); Akita University, Akita-shi Akita 010-8502 (JP)
(72) Inventor: SHIOTA Akira, Tokyo 112-0004 (JP); MIZOROKI Tatsuya, Utsunomiya-shi Tochigi 321-0973 (JP); MORITOKI Yuki, Akita-shi Akita 010-8502 (JP)
(74) Representative: D Young & Co LLP
(86) International application number: PCT/JP2016/063356
(87) International publication number: WO 2016/175285

(56) References cited:
- WO-A2-2010/039900
- JP-A- 2005 528 918
- JP-A- 2006 513 725
- JP-A- 2006 517 096
- JP-A- 2008 154 468
- JP-A- 2013 514 778
- US-A1- 2006 218 655
- US-A1- 2011 154 512
- REILLY M P ET AL: "HEPARIN-INDUCED THROMBOCYTOPENIA/THROMBOSIS IN A TRANSGENIC MOUSE MODEL REQUIRES HUMAN PLATELET FACTOR 4 AND PLATELET ACTIVATION THROUGH FCGAMMARIIA", BLOOD, AMERICAN SOCIETY OF HEMATOLOGY, US, vol. 98, no. 8, 15 October 2001 (2001-10-15), pages 2442-2447, XP001035093, ISSN: 0006-4971, DOI: 10.1182/BLOOD.V98.8.2442
- Anonymous: "Preclinical safety evaluation of biotechnology-derived pharmaceuticals", , 1 January 2011 (2011-01-01), XP055499321, Retrieved from the Internet: URL:http://www.ema.europa.eu/docs/en_GB/do cument_library/Scientific_guideline/2009/0 9/WC500002828.pdf [retrieved on 2018-08-13]

### SEQUENCE LISTING

<110> Institute of Immunology Co., Ltd.
<120> A transgenic non-human animal expressing a specific human molecule and human Fc gamma receptor family
<130> PH-6540-PCT
<150> JP 2015-093445
   <151> 2015-04-30
<160> 6
<170> PatentIn version 3.5
<210> 1
   <211> 50
   <212> DNA
   <213> Artificial
<220>
   <223> primer
<400> 1
   atgacaacac ccagaaattc agtaaatggg actttcccgg cagagccaat 50
<210> 2
   <211> 50
   <212> DNA
   <213> Artificial
<220>
   <223> primer
<400> 2
   ctccccaaga tcaggaatcc tcaccaatag aaaatgacag ctctccttaa 50
<210> 3
   <211> 50
   <212> DNA
   <213> Artificial
<220>
   <223> primer
<400> 3
   cctagacatt tagactagat agcaagatgt tttggaaagc aagaggcagc 50
<210> 4
   <211> 50
   <212> DNA
   <213> Artificial
<220>
   <223> primer
<400> 4
   aggaacatcc acttccatct accccttctt gcttacaatt ctgtttggtt 50
<210> 5
   <211> 50
   <212> DNA
   <213> Artificial
<220>
   <223> primer
<400> 5
   aagactctga tctcacctca ctgtcttatt ttcaggcgtt tgaaaactca 50
<210> 6
   <211> 50
   <212> DNA
   <213> Artificial
<220>
   <223> primer
<400> 6
   actcttttct tttctaagca ttattgttta gagagcttcc aagacacata 50

## Description

### TECHNICAL FIELD

The present invention relates to a transgenic non-human animal, which expresses the genes encoding human CD20, and the human Fcγ receptor family members, and to a method for evaluating properties, safety and therapeutic effect of an antibody which binds to human CD20 and has all or part of a peptide sequence of a human-derived gamma chain Fc region *in vivo,* using the transgenic non-human animal.

### BACKGROUND ART

The significant progress in genetic engineering techniques or antibody engineering techniques in recent years has enabled development of targeted drugs to human molecules, including antibody drugs as typical examples. However, it is impossible to make an appropriate evaluation of a pharmacological effect or safety of such a targeted drug to a human molecule in a preclinical animal study, because non-human animals used for preclinical studies (e.g., mouse, rat, rabbit, or the like) do not express a human antigen reacting with the administered molecular targeted drug. Accordingly, in the development of molecular targeted drugs such as a human chimeric antibody and a humanized antibody, it has been desired to develop a method for evaluating the safety or therapeutic effects of such molecular targeted drugs by *in vivo* animal experiments (method of preclinical study) before clinical trials.

Now that the Human Genome Project is completed, it is easy to acquire bioresources encoding genome information of human molecules used for target antigens and its genome fragments. Therefore, hundreds of molecular targeted drug projects, especially antibody drug projects, have been launched and engaged in fierce competition between biotech companies. Because of high specific binding of a monoclonal antibody to an antigen (a target molecule), currently developed antibody drugs exert a high safety and low toxicity. In addition, methods for producing such monoclonal antibodies have already been well established and are relatively easy. Moreover, once an antibody having excellent specificity and binding activity is obtained, the activity can be enhanced by a variety of modifications. However, not all of such efforts improve clinical outcomes significantly, and modified antibody drugs often need large doses, which results in high drug prices. One of the reasons for the aforementioned problems is that there are no appropriate animal models, which can be used to evaluate the bioactivity of an antibody against a human antigen *in vivo,* and thus the antibody drug is not tested in disease model animals in a preclinical stage. In order to solve this problem, it is essential to produce an animal model for use in evaluating *in vivo* the bioactivity of a molecular targeted drug including an antibody as a representative example, and thus, it has been desired to develop an evaluation method, in which an appropriate animal model is used.

According to 1st Chapter, 3. Non-Clinical Safety Test, 3.3 Selection of Animal Species/Models of Notification from the Director of Pharmaceutical and Food Safety Bureau, Ministry of Health, Labour and Welfare, "Biotechnology Oyo Iyakuhin no Hirinsho niokeru Anzensei Hyoka (Preclinical Safety Evaluation of Biotechnology-Derived Pharmaceuticals)," (Notification No. 0323-1 of PBA, dated March 23, 2012) (Non Patent Literature 1), it is described that "Relevant animal species for testing of monoclonal antibodies are those that express the desired epitope and demonstrate a similar tissue cross-reactivity profile as for human tissues. " It is also described therein that "When no relevant species exists, the use of relevant transgenic animals expressing the human receptor or the use of homologous proteins should be considered", which means utilization of transgenic animals is proposed. Moreover, it is further described that "The information gained from use of a transgenic animal model expressing the human receptor is optimized." On the other hand, the Notification also proposes utilization of an evaluation method of using non-human primates such as a rhesus monkey having the aforementioned homologous protein (i.e., antigen homolog) similar to a human antigen. However, animal experiments using non-human primates are extremely restricted from the ethical and welfare viewpoints, and extraordinarily expensive and thus cannot be easily done at the drug discovery stage. Accordingly, it has been desired to develop an evaluation method by using transgenic animals, instead of non-human primates.

Under such circumstances, many studies regarding transgenic mice, expressing a human antigen, a human receptor, or the like recognized by a monoclonal antibody, have been published, which purposes are utilization of such transgenic mice in development of antibody drugs. On the other hand, when the transgenic animals are produced by pronuclear injection of a small plasmid-type expression vector, it is well known that such transgenic animals don't reproduce the desired expression level and the native precise expression control of the introduced human gene. Hence, it has been reported that an large size of human genome-derived DNA fragment harboring a gene encoding a human antigen, a human receptor, or the like, as well as a sequence for controlling the expression of the human gene, has been used as an expression vector (or a BAC expression vector), and that the expression vector has been then introduced into fertilized eggs by pronuclear injection to produce a transgenic mouse, and according to this method, transgenic mice expressing human CD20 have been actually produced (Patent Literature 1, Patent Literature 3 and Non Patent Literature 2). In those transgenic mice, CD20 is expressed on the mature B cells, pre B cells, and immature B cells in blood, bone marrow, spleen, lymph nodes and Peyer's patches. However, the expression level of human CD20 on these cells has been approximately 40% of the expression level of CD20 on human cells (Patent Literature 1). In addition, the expression level of human CD20 on the spleen or B cells of such a transgenic mouse has been at most seven times lower than the expression level of human CD20 in a human (Non Patent Literature 2). Moreover, a knock-in mouse which genome sequence is modified to co-express human CD16 and its accessory molecules thereof, was reported as a non-human animal for reproducing the human immune system (Patent Literature 2). It has been also reported that, when the thus produced transgenic mouse expressing a human antigen is used, an antibody against the human antigen exhibits pharmacological activity even *in vivo* (Non Patent Literature 2 and Non Patent Literature 3). In addition, genetically modified mice, and methods and compositions for making and using them have been reported, wherein the mice lack endogenous murine FcyR genes, including genetically modified mice that comprise a replacement of the endogenous murine FcyR genes with human FcyR genes, and including mice that are capable of expressing up to five functional human low-affinity FcyR-chain genes (Patent Literature 4).

However, though the pharmacological effects of an antibody drug has been evaluated *in vivo* with the above-described transgenic mice which express a human antigen, the effective doses of the antibody in such studies were eventually much more than the clinical dose, and as a result, it has caused a problem to the validity of the evaluation (Non Patent Literature 2 and Non Patent Literature 4).

As described above, in the development of molecular targeted drugs including antibody drugs as typical examples, there have been no appropriate animal models that can be used to evaluate *in vivo* the bioactivity, efficacy or safety of a drug, and also, there have been no means for non-clinical tests of using pathogenic models. Therefore, it has been desired to develop a novel method capable of appropriately evaluating drugs, using animal models.

### CITATION LIST

### Patent Literature

Patent Literature 1: JP Patent Publication (*Kohyo*) No. 2006-517096 A
Patent Literature 2: JP Patent Publication (*Kokai*) No. 2008-154468 A
Patent Literature 3: US patent publication No. 2006/218655 A1
Patent Literature 4: US patent publication No. 2011/154512 A1

### Non Patent Literature

Non Patent Literature 1: *"*Biotechnology Oyo Iyakuhin no Hirinsho niokeru Anzensei Hyoka (Preclinical Safety Evaluation of Biotechnology-Derived Pharmaceuticals)," (Notification No. 0323-1 of PBA, dated March 23, 2012)
Non Patent Literature 2: Ahuja A.et al., 2007, J. Immunol. 179: 3351-3361
Non Patent Literature 3: Stephen A. et al., 2008, Blood 112: 4170-4177
Non Patent Literature 4: Huang H. et al., 2010, PNAS 107: 4658-4663

### SUMMARY OF INVENTION

### Technical Problem

It is an object of the present invention to provide a transgenic non-human animal capable of appropriately evaluating properties, pharmacological effect and safety of a molecular targeted substance or a targeted drug to a human specific molecule.

### Solution to Problem

As a result of intensive studies directed towards achieving the aforementioned object, the present inventors have found that three or more copies of a human CD20 recombinant BAC expression vector and the BAC expression vector for genes encoding human Fcγ receptor family members are introduced by pronuclear injection into a non-human animal used as a host, such as mouse, rat or rabbit, to produce a transgenic non-human animal that expresses the genes encoding human CD20 and the genes encoding human Fcγ receptor family members, and the transgenic non-human animal is then utilized, so that the properties, pharmacological effect, safety and the like of a molecular targeted substance or a targeted drug to the human specific molecule can be evaluated under a situation close to the original living body of a human, thereby completing the present invention.

The present invention is described in the appended claims.

Specifically, the present invention includes the following:
[1] A transgenic non-human animal, into which three or more copies of a human CD20 recombinant BAC expression vector and the BAC expression vector for genes encoding human Fcγ receptor family members have been introduced, and which expresses the genes encoding human CD20 and the genes encoding human Fcγ receptor family members, wherein:
   (i) the human Fcγ receptor family members comprise human FcyRIIa, human FcyRIIb, human FcyRIIc, human FcyRIIIa, and human FcyRIIIb;
   (ii) the genes encoding human CD20 are expressed through the mechanism that controls the expression of the homolog gene of the host non-human animal to the gene encoding human CD20; and
   (iii) the transgenic non-human animal is selected from a mouse, rat, hamster, guinea pig, rabbit, dog, cat, bovine, sheep, horse, or swine.
[2] A method for evaluating a property of a test substance, comprising: administering a test substance to the transgenic non-human animal according to the above [1]; and then evaluating *in vivo* the property of the test substance in the transgenic non-human animal, wherein the test substance is an antibody which binds to human CD20 and has all or part of a peptide sequence of a human-derived gamma chain Fc region.
[3] The method according to the above [2], wherein the property of the test substance is an ability to inhibit the function of human CD20, an ability to bind to humanCD20, a cytotoxic activity to cells, tissues or organs expressing humanCD20, biodistribution, pharmacological effects, or safety.

### EFFECT OF INVENTION

According to the present invention, there can be provided: a transgenic non-human animal according to the above [1] which is capable of high-sensitively and appropriately evaluating properties, pharmacological effect, safety, and the like of an antibody which binds to human CD20 and has all or part of a peptide sequence of a human-derived gamma chain Fc region.

### BRIEF DESCRIPTION OF DRAWINGS

[Fig. 1] This figure shows the schematic view of a BAC expression vector comprising the gene cluster of the human Fcγ receptor family.
[Fig. 2] This figure shows the schematic view of a BAC clone comprising the mouse CD20 gene locus.
[Fig. 3] This figure shows the schematic view of a BAC clone comprising the human CD20 gene locus.
[Fig. 4] This figure is the schematic view showing a method of constructing recombinant BAC expression vectors for human CD20 gene.

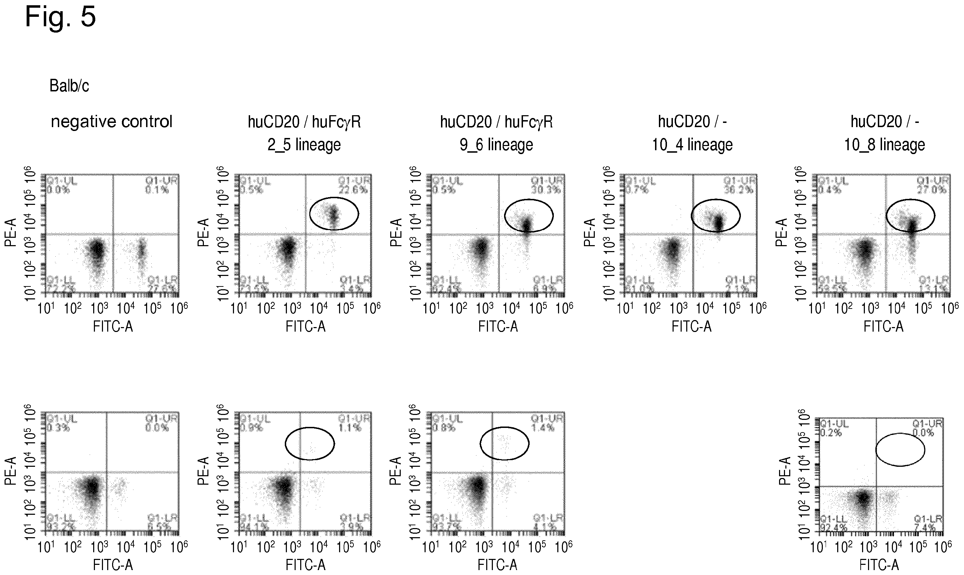

[Fig. 5] This figure shows the detection results of human CD20-expressing B cells (upper panels) and human FcyRIIIa-expressing NK cells (lower panels), in individual lineages of CD20 human antigen mice (huCD20/huFcyR) and transgenic mice (huCD20/-) into which only a human CD20 recombinant BAC expression vector has been introduced, both of which had been obtained from Balb/c mice. In each panel on the upper panels, the longitudinal axis indicates PE-human CD20, while the horizontal axis indicates FITC-CD45R/B220 (B cells. In each panel on the lower panels, the longitudinal axis indicates PE-human CD16 (FCyR), while the horizontal axis indicates FITC-pankNK (NK cells). Individual cells indicating individual antigens are detected in each region surrounded with the circle.

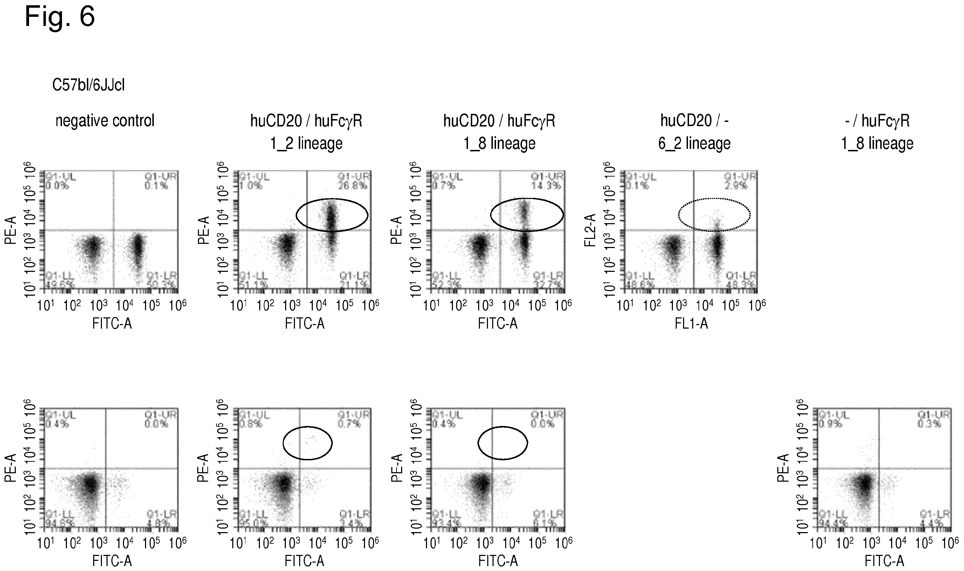

[Fig. 6] This figure shows the detection results of human CD20-expressing B cells (upper panels) and human FcyRIIIa-expressing NK cells (lower panels), in individual lineages of CD20 human antigen mice (huCD20/huFcyR) and transgenic mice (huCD20/-) into which only a human CD20 recombinant BAC expression vector has been introduced, both of which had been obtained from C57bl/6JJcl mice. In each panel on the upper panels, the longitudinal axis indicates PE-human CD20, while the horizontal axis indicates FITC-CD45R/B220 (B cells. In each panel on the lower panels, the longitudinal axis indicates PE-human CD16 (FCyR), while the horizontal axis indicates FITC-pankNK (NK cells). Individual cells indicating individual antigens are detected in each region surrounded with the circle.
[Fig. 7] This figure shows the measurement results of the number of splenic B lymphocytes in CD20 human antigen mice, to which various types of therapeutic anti-CD20 antibody drugs have been administered.
[Fig. 8] This figure shows the results measured with time of the number of splenic B lymphocytes in CD20 human antigen mice, to which various types of therapeutic anti-CD20 antibody drugs have been administered.
[Fig. 9] This figure shows the measured results of the number of splenic B lymphocytes in (A) transgenic mice into which only a human CD20 recombinant BAC expression vector has been introduced (10_8 lineage (huCD20+/huFcγR-)) and (B) CD20 human antigen mice (9_6 lineage (huCD20+/huFcγR+)), to both of which various types of therapeutic anti-CD20 antibody drugs have been administered. Each graph is shown with a relative value to the value of the number of splenic B lymphocytes in a control group, which is defined as 100%.
[Fig. 10] This figure shows the results measured with time of the number of B lymphocytes in peripheral blood, after a therapeutic anti-CD20 antibody drug has been administered to CD20 human antigen/disease model mice obtained by crossing CD20 human antigen mice with genetically modified primary biliary cirrhosis model mice.
[Fig. 11] This figure shows the measured results of (A) the total number of lymphocytes, (B) the number of CD8-positive cytotoxic T cells, and (C) the number of CD44-highly expressing and CD8-positive activated cytotoxic T cells, which infiltrated into the liver of the above-described CD20 human antigen/disease model mice, to which a therapeutic anti-CD20 antibody drug has been administered.
[Fig. 12] This figure shows (A) severity level, (B) severity frequency, and (C) severity score of the substantial inflammatory area in the liver of the above-described CD20 human antigen/disease model mice, to which a therapeutic anti-CD20 antibody drug has been administered.

### DESCRIPTION OF EMBODIMENTS

### 1. Transgenic non-human animal

In the present invention, the "human specific molecule" is human CD20. In the present disclosure, the "human specific molecule" includes, for example, a protein or a polypeptide that is expressed in human cells, or mutants thereof. The human specific molecule is preferably any given human antigen molecule that may be a target of a molecular targeted drug (e.g., an antibody drug) or the like. The human specific molecule is more preferably associated with a human disease or disease symptoms, namely, the human specific molecule may be a causative factor for the disease or disease symptoms. Herein, the "mutant" means: a protein or a polypeptide, which has an amino acid sequence comprising a substitution, deletion, addition or insertion of one or more amino acids in the amino acid sequence of the original protein or polypeptide, and which retains the activity or function of the original protein or polypeptide; or a protein or a polypeptide, which has a sequence identity of 80% or more, 90% or more, 95% or more, 97% or more, or 99% or more with all or part of the amino acid sequence of the original protein or polypeptide, and retains the activity or function of the original protein or polypeptide. The sequence identity of an amino acid sequence can be obtained by a known method of comparing amino acid sequences, and for example, such comparison can be carried out using BLAST (Basic Local Alignment Search Tool at the National Center for Biological Information, U.S.A) or the like at default settings.

Preferably, in the present disclosure, examples of the "human specific molecule" include human CD20, epidermal growth factor receptor (EGFR), CD3, insulin-like growth factor-I receptor (IGF-IR), human epidermal growth factor receptor 2 (HER2), CD40, CD19, CD326, CD25, CD4, CD45, CD22, CD30, cytotoxic T-lymphocyte-associated protein 4 (CTLA4), GM-2, intercellular adhesion molecule 1 (ICAM1), cancer antigen 125 (CA125), CD52, programmed death-1 (PD-1), B-cell activating factor (BAFF), C-C chemokine receptor type 4 (CCR4), vascular endothelial growth factor receptor-3 (VEGFR-3), CD248, C-X-C motif chemokine 10 (CXCL10), C-C motif chemokine 2 (CCL2), interleukin 6 receptor (IL-6R), GD2, delta-like ligand 4 (DLL4), C-C chemokine receptor type 5 (CCR5), tumor-associated glycoprotein 72 (TAG-72), CD38, programmed death-ligand 1 (PD-L1), T cell immunoglobulin mucin-3 (TIM-3), lymphocyte activation gene 3 (LAG-3), T cell immunoglobulin and ITIM domain (TIGIT), CD33, CD137, vascular endothelial growth factor receptor-2 (VEGFR-2), CD2, B7-H3, B7-H4, and B7-H5. Among them, human CD20 is particularly preferable.

In the present invention, the "human Fcγ receptor family" means a series of human Fcγ receptors, which are expressed in, what are called, human effector cells, such as killer T cells, helper T cells, regulatory T cells, natural killer cells, or macrophages. The human Fcγ receptors consist of the family of the five receptors, FcγRIIa, IIb, IIc, IIIa and IIIb. Among them, FcγRIIa, FcγRIIc, FcγRIIIa and FcγRIIIb are called an activation type of Fcγ receptors, and bind to a conjugate of antigen and IgG antibody (i.e., an IgG immune complex), so as to activate effector cells. On the other hand, FcγRIIb is called a suppression type of Fcγ receptor, and binds to the IgG immune complex, so as to suppress the activation of effector cells. In the present invention, the "human Fcγ receptor family" comprises the above-described FcγRIIa, FcγRIIIa, FcγRIIc, FcγRIIIb, and FcγRIIb. A group of genes encoding these Fcγ receptors forms the cluster in 1q23 of human chromosome 1 and is present therein. In addition, in the present invention, the "human Fcγ receptor family" includes mutants of the Fcγ receptors. The term "mutant" is used herein to mean: a protein or a polypeptide, which has an amino acid sequence comprising a substitution, deletion, addition or insertion of one or more amino acids in the amino acid sequences of one or more (e.g., 2, 3, 4, 5, 6, 7, or 8) proteins or polypeptides included in the human Fcγ receptor family, and retains the activity or function of the original protein or polypeptide; or a protein or a polypeptide, which has a sequence identity of 80% or more, 90% or more, 95% or more, 97% or more, or 99% or more with all or part of the amino acid sequence of the original protein or polypeptide, and which retains the activity or function of the original protein or polypeptide.

In the present invention, the amino acid sequences of the human specific molecules and the human Fcγ receptor family, or the nucleotide sequences of genes encoding them, are disclosed in known databases including GenBank, and so, in the present invention, such sequence information is available. For example, the amino acid sequence of human CD20 and the nucleotide sequence encoding it have been registered under the number BC002807 with GenBank. With regard to the human Fcγ receptor family, the amino acid sequences of FCγRIIa, FCγRIIIa, FCγRIIc, FCγRIIIb and FCγRIIb, and the nucleotide sequences of genes encoding them have been registered with GenBank under the numbers BC019931, BC017865, BC137397, BC128562, and BC031992, respectively, and so, in the present invention, such sequence information is available.

In the present invention, the "non-human animal" is selected from mouse, rat, hamster, guinea pig, rabbit, dog, cat, bovine, sheep, horse, or swine. The non-human animal is preferably mouse or rat in which the basis of the genetic engineering techniques has been established, and which is easily available. For instance, in the present invention, when the non-human animal is a mouse, C57BL/6 or BALB/c can be used, and when the non-human animal is a rat, Wistar rats, Sprague Dawley rats, etc. can be used.

In the present invention, the "homolog gene of a non-human animal" means a gene, which is possessed by a non-human animal having the same evolutionary origin as a gene encoding the human specific molecule or the human Fcγ receptor family, and which encodes a protein having a structure or function equal to or similar to the human specific molecule or the human Fcγ receptor family. In general, a human gene and a homolog gene (or an orthologous gene) of a non-human animal have high sequence identity of, for example, 80% or more, 90% or more, 95% or more, 97% or more, or 99% or more. For instance, a mouse orthologous gene of human CD20 has been known and has been registered with GenBank under the number BC028322, and also, mouse orthologous genes of the human Fcγ receptor family have been known and have been registered with GenBank under the numbers of BC038070 and BC027310. In the present invention, such sequence information is available.

In the present invention, the "transgenic non-human animal" includes not only a transgenic non-human animal produced by the below-mentioned procedures and the lineages thereof, but also their progeny, and portions thereof. The term "progeny" used herein means a non-human animal, which is obtained by crossing a non-human animal with the transgenic non-human animal of the present invention or a lineage thereof, and which has genes encoding a human specific molecule and genes encoding a human Fcγ receptor family, and expresses the genes encoding a human specific molecule and the genes encoding a human Fcγ receptor family. Moreover, the term "portions" means organs, tissues and cells derived from the transgenic non-human animal of the present invention, lineages thereof, and progeny thereof.

Disclosed herein, the transgenic non-human animal of the present disclosure can be produced by standard methods for producing BAC transgenic non-human animals, which have been generally used in the technical field. That is to say, the transgenic non-human animal of the present disclosure can be generally produced by a method comprising the following steps (a) to (c):
(a) a step of introducing a BAC expression vector that comprise a BAC comprising the gene encoding a human specific molecule and a BAC expression vector that comprises a BAC comprising the gene encoding a human Fcγ receptor family, into the fertilized eggs of a non-human animal used as a host, and then transplanting the gene-introduced fertilized eggs into surrogate animals; and
(b) a step of selecting a transgenic non-human animals having the gene encoding a human specific molecule and the gene encoding a human Fcγ receptor family, from the offsprings (or progeny) born from the surrogate animals obtained in the step (a); and
(c) a step of establishing a lineage from the transgenic non-human animal (founder) selected in the step (b).

Hereafter, the individual steps will be described.

In the above-described step (a), the BAC comprising a gene encoding a human specific molecule and the BAC comprising a gene encoding a human Fcγ receptor family mean the bacterial artificial chromosome (BAC) comprising DNA encoding a human specific molecule or genomic DNA, and the bacterial artificial chromosome (BAC) comprising DNA encoding a human Fcγ receptor family or genomic DNA, respectively. Each BAC comprises DNA encoding a human specific molecule or genomic DNA, DNA encoding a human Fcγ receptor family or genomic DNA, and sequences that influence the transcription, translation and stability of such DNAs (i.e., the 5'-flanking region and/or the 3'-flanking region).

With regard to the BAC comprising a gene encoding a human specific molecule and the BAC comprising a gene encoding a human Fcγ receptor family, known human genome database (e.g., database disclosed by University of California, Santa Cruz) can be searched, and human BAC clones including predetermined genes can be selected and/or used. With regard to the BAC comprising a gene encoding a human specific molecule, a plurality of human BAC clones each comprising a gene encoding a different human specific molecule may also be used.

In the present disclosure, the obtained human BAC clones can be each used as BAC expression vectors. Preferably, an expression vector produced by substituting a homolog gene (or orthologous gene) of a BAC clone comprising the homolog gene of a non-human animal used as a host by a predetermined gene of a human BAC clone (hereinafter referred to as a "recombinant BAC expression vector") is used. A human gene derived from a human BAC clone, which has been introduced into a non-human animal via the recombinant BAC expression vector, may be expressed in the non-human animal according to the mechanism for controlling the expression of the homolog gene. The expression of the homolog gene in the non-human animal is considered to have the correlation with the expression of the corresponding human gene in the living human body (i.e., the site (organ or tissue) of the living human body, in which the corresponding human gene is expressed, corresponds to the site (organ or tissue) of the living non-human animal body, in which the homolog gene to the human gene is expressed), and thus, the introduced human gene can be expressed in the non-human animal, in the manner (i.e., organ or tissue) that is close to the original living human body because the expression of a gene encoding the human specific molecule is directed by a same controlling mechanism as the expression of an orthologous gene of the host non-human animal.

Upon production of a recombinant BAC expression vector, with regard to BAC clones comprising an orthologous gene of a non-human animal, known human genome database (e.g., database disclosed by University of California, Santa Cruz) can be searched, and the BAC clone of a non-human animal comprising a predetermined orthologous gene can be selected and/or used. When a plurality of BAC clones comprising the genomic sequence of the corresponding orthologous gene are present in a non-human animal used as a host, a BAC clone including a long 5'-flanking region and/or 3'-flanking region is selected, so that precise expression of the introduced human gene can be preferably realized in the transgenic non-human animal.

A predetermined gene of a human BAC clone can be substituted with a orthologous gene of the corresponding non-human animal by accurately removing a region encoding a start codon to a stop codon of the orthologous gene from the BAC of the non-human animal, and then inserting a region encoding a start codon to a stop codon of a predetermined gene of a human BAC clone into the removed region. For production of a recombinant BAC expression vector, for example, a BAC homologous recombination method that involves a Red system using a defective lambda prophage can be utilized. Upon production of such a recombinant BAC expression vector, it is preferable to completely remove antibiotic-resistant genes derived from *Escherichia coli,* which have been inserted during the recombination process.

In the present disclosure, at least, the gene encoding a human specific molecule preferably is in the form of a recombinant BAC expression vector.

The BAC expression vector, as well as the recombinant BAC expression vector, is preferably in the purified form. The BAC expression vector can be purified, for example, by applying common means for purifying nucleic acids, and for example, the BAC expression vector, which has been linearized with suitable restriction enzymes, is separated by pulsed-field electrophoresis and is then subjected to electric elution, so that it can be purified.

As a method of introducing each BAC expression vector into the fertilized eggs of a non-human animal used as a host, for example, a method which comprises washing the oviduct of female animals immediately after crossing to collect fertilized eggs being in the pronuclear stage, and then directly injecting the BAC expression vector into the sperm- or egg-derived pronucleus, preferably into the male pronucleus, by the microinjection method (the so-called microinjection method). Individual BAC expression vectors may be injected into the pronucleus, simultaneously or successively. The fertilized eggs are transplanted into the oviduct of pseudopregnant surrogate female animals, and the development is continued in the uterus.

In the step (b), a transgenic non-human animal can be selected from offsprings born from the animal, into which the fertilized egg has been transplanted in the step (a), by detecting whether or not the gene encoding a human specific molecule and the gene encoding a human Fcγ receptor family have been integrated into the chromosomal DNA of the obtained offsprings. The detection can be carried out by conventional methods, for example, by screening chromosomal DNA separated and extracted from the tail portion of the offsprings according to the Southern blotting method or the PCR method.

In the step (c), as a method of establishing a lineage having the same genetic background from the transgenic non-human animal (founder) selected in the step (b), there may be a method which comprises backcrossing an animal into which the gene encoding a human specific molecule has been incorporated, with a wild-type animal (the C57BL/6 or BALB/c mouse, the Wistar or Sprague Dawley rat, or the like).

Unlike wild-type animals of the same species, the transgenic non-human animal of the present invention can be an animal, to which any of high-molecular-weight and low-molecular-weight compounds interacting with a human specific molecule can be administered, and which can be then used to evaluate biological responses, and thus, the present transgenic non-human animal can become an animal model, which can greatly contribute to the progress of molecular targeted therapy including evaluation of the pharmacological effects of molecular targeted drugs and evaluation of safety. In recent years, in order for an antibody drug to exhibit pharmacological activity, effector activity is considered to be important, and evaluation of the pharmacological effects of molecular targeted drugs including such effector activity, evaluation of safety, and the like are required. On the other hand, since the conventionally known transgenic mice expressing human antigens (Ahuja A.et al., 2007, Huang H.et al., 2010, *ibid*) do not have a human Fcγ receptor(s), an antibody drug administered to the mice cannot sufficiently exhibit its effector activity, and thus, there is a case where validity is insufficient in evaluation of the pharmacological effects of the drug, evaluation of the safety thereof, and the like. Since the transgenic non-human animal of the present invention has a human Fcγ receptor family, it can reproduce biological responses including effector activity, and the administered antibody drug can be evaluated in a situation close to the inside of the living body of the original human.

Moreover, the transgenic non-human animal of the present invention can be produced by applying the microinjection method, and this method does not need complicated operations like the gene targeting method that is also commonly used for introduction of a human gene into non-human animals. As such, the transgenic non-human animal of interest can be quite simply and quickly obtained.

Furthermore, the transgenic non-human animal of the present invention can be crossed with another animal disease model of the same species, so that a non-human animal disease model expressing a human specific molecule and a human Fcγ receptor family can be obtained. Examples of such "another animal disease model" include known animal models (e.g., NOD mouse (i.e., type I diabetes having mouse model)) having diseases such as type I diabetes and autoimmune diseases (i.e., rheumatoid arthritis, lupus nephritis, systemic lupus erythematosus, and the like).

Disclosed herein, upon production of the transgenic non-human animal of the present disclosure, it is also possible to allow a mutant human specific molecule to express by utilizing a gene comprising a genetic mutation that provides an amino acid substitution to the amino acid sequence of the human specific molecule (i.e., a mutant gene). Thus, it is also possible to produce a transgenic non-human animal that expresses a mutated human specific molecule and is highly sensitive or resistant to a molecular targeted drug. For instance, if transgenic non-human animals expressing mutant human specific molecules (EGFR, KRAS, HER2, BRAF, DDR2, etc.) and having resistance to molecular targeted drugs were produced, the produced transgenic non-human animals could be utilized to produce molecular targeted drugs exhibiting sensitivity to non-human animal models having such resistance. In the present disclosure, the transgenic non-human animal that expresses such mutated human specific molecule as well as a human Fcγ receptor family can also be provided.

### 2. Screening/evaluation methods

### (2-1) Method for evaluating properties

The present disclosure provides a method for evaluating a property or properties of a test substance, which comprises the following steps of:
(a) administering a test substance to the above-described transgenic non-human animal of the present disclosure; and
(b) evaluating *in vivo* the property or properties of the test substance in the above-described transgenic non-human animal, to which the test substance has been administered.

In the step (a), the test substance may be any type of molecular targeted substance. Examples of the test substance include nucleic acids, carbohydrates, lipids, proteins, peptides, organic low-molecular-weight compounds, compound libraries produced using combinatorial chemistry techniques, antibodies against human specific molecules, aptamers, random peptide libraries produced by the solid-phase synthesis or phage display method, natural components derived from microorganisms, animals, plants, marine organisms, etc., and mixtures consisting of two or more types of these compounds. In addition, the test substance can also include a drug(s) comprising, as an active ingredient(s), one or more of these compounds. For example, in the present invention, an antibody is used as such a test substance. The antibody may be either a polyclonal antibody or a monoclonal antibody, and it may also be a human chimeric antibody or a humanized antibody.

The route of administration of the above-described test substance to the transgenic non-human animal of the present invention is not particularly limited. The test substance can be orally or parenterally administered. Examples of the parenteral administration route include systemic administration such as intravenous, intra-arterial or intramuscular administration, and local administration such as administration to around target cells.

The administration dose of the above-described test substance can be appropriately determined depending on the type of active ingredient, the size of molecule, administration route, animal species used as a subject to be administered, acceptability of the drug, body weight and age of such subject, and the like.

In the step (b), examples of the property or properties of the test substance include an ability to bind to a human specific molecule, an ability to inhibit the function of a human specific molecule, an cytotoxic activity to cells, tissues or organs expressing a human specific molecule, biodistribution, pharmacological effects, or safety.

As a method for evaluating the property or properties, general procedures for quantifying proteins or nucleic acids, can be used. The evaluation method is, for example, a method which comprises collecting predetermined organs or tissues from the transgenic non-human animal of the present invention before and after administration of a test substance, then quantifying the expression level of a human specific molecule or a gene encoding it in the organs or tissues, or the expression level of a molecule located downstream of a signal cascade with which the human specific molecule is associated, or a gene encoding it, or the content of the test substance by using flow cytometry, Western blotting, RT-PCR, real-time PCR, etc., and then analyzing a change in the expression level or the content before and after administration of the test substance. In addition to or instead of analyzing a change in the aforementioned expression level or content, the other examples of the evaluation method include a method which comprises quantifying the titer of an antibody against a human specific molecule by the ELISA method, and a method of analyzing the enhancement or suppression of phosphorylation of a human specific molecule or the enhancement or suppression of a downstream signal, at a protein level, according to immunoprecipitation.

### (2-2) Screening method

The present disclosure provides a method for screening and/or evaluating a candidate substance for treating a disease, which comprises the following steps of:
(a) administering a test substance to the above-described transgenic non-human animal of the present disclosure;
(b) comparing the extent of the disease in the transgenic non-human animal to which the above-described test substance has been administered, with the extent of the disease in a control to which the above-described test substance has not been administered; and
(c) selecting a test substance that has or is expected to have a more desired effect (or efficacy), or evaluating the pharmacological effects/safety of the test substance, based on the above-described comparative results.

In the above-described step (a), the test substance and the administration method are the same as those described in the above-described method for evaluating the property or properties.

In the step (b), as a method of examining the extent of the disease in the animal to which the test substance has been administered, the analysis method described in the aforementioned method for evaluating the property or properties can be applied. A control animal, to which the test substance has not been administered, can also be examined in the same manner.

In the step (c), based on the comparative results obtained in the step (b), a test substance for enhancing or inhibiting the function or activity of a human specific molecule is selected, or the pharmacological effects/safety of the test substance is evaluated. With regard to selection/evaluation criteria, the results of a control animal to which the test substance has not been administered may be used as indicators.

The thus evaluated or selected test substance has an effect (or an action) to improve a disease caused by the enhancement or inhibition of the function or activity of a human specific molecule, and thus, the test substance can be a candidate drug for treating the disease.

Disclosed herein is a method for screening for a candidate substance(s) for treating autoimmune diseases, which comprises the following steps of:
(a) administering a human CD20-targeted antibody as a test substance to the above-described transgenic non-human animal of the present disclosure expressing the human Fcγ receptor family and the human CD20 as a human specific molecule;
(b) collecting human CD20-expressing lymphocytes from the above-described transgenic non-human animal, to which the above-described antibody has been administered, and evaluating them by flow cytometry, and/or collecting serum and quantifying the titer of an autoantibody by the ELISA method, and/or confirming suppression of phosphorylation of a human antigen or suppression of a downstream signal, at a protein level, according to Western blotting and immunoprecipitation, and comparing the obtained results with the results of a control to which the antibody has not been administered; and
(c) selecting an antibody capable of suppressing the activation of lymphocytes and suppressing generation of an autoantibody, as a candidate substance for treating an autoimmune disease, based on the above-described comparative results.

More specifically, the present disclosure provides a method for screening for a candidate substance for treating a disease having resistance to known molecular targeted drugs, which comprises the following steps of:
(a) administering a test substance and a known molecular targeted drug (Gefitinib, Erlotinib, Selumetinib, Afatinib, Neratinib, Vemurafenib, Dasatinib, Imatinib, or the like) to transgenic non-human animals that express a mutant human specific molecule (EGFR, KRAS, HER2, BRAF, DDR2, or the like) associated with resistance to the molecular targeted drug and a human Fcγ receptor family;
(b) collecting organs or tissues expressing the mutant human specific molecule from the animals, to which the above-described test substance has been administered, and then quantifying the expression level of a mutant human specific molecule or a gene encoding it in the organs or tissues, or the expression level of a molecule located downstream of a signal cascade with which the human specific molecule is associated or a gene encoding it, at the level of a gene and/or a protein, or quantifying the titer of an antibody against the human specific molecule, and then comparting the obtained results, with the quantification results of a control to which the test substance has not been administered; and
(c) selecting a test substance capable of enhancing sensitivity to the molecular targeted drug as a candidate substance for treating the above-described disease, based on the above-described comparative results.

The thus selected test substance has an effect (or an action) to improve a disease caused by the enhancement or inhibition of the function or activity of the above-described mutant human specific molecule, and thus, the test substance can be a candidate substance for treating a disease having resistance to known molecular targeted drugs.

Hereinafter, the present invention will be described in detail, referring to the following examples. However, these examples are used for illustrative purpose only, and they are not intended to limit the scope of the present invention.

### EXAMPLES

### Example 1: Selection of BAC comprising gene cluster of human Fcγ receptor family

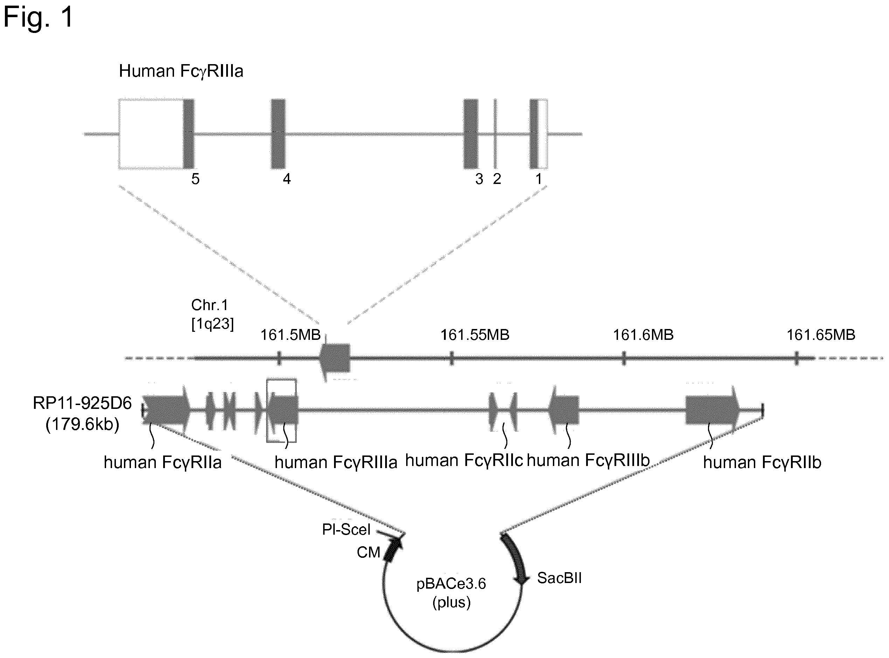

Based on the cDNA sequences of human FcyRIIa, IIb, IIc, IIIa and IIIb genes (GenBank: BC019931, BC031992, BC137397, BC017865 and BC128562), human genomic sequences were searched by using UCSC Genome Browser, and a BAC clone including the gene cluster encoding all receptors of the human Fcγ receptor family was screened for. As a result, RP11-925D6 with a size of 179.6 kb, which included all of the genomic DNA sequences of the human FcyRIIa, IIb, IIc, IIIa and IIIb genes, was identified and acquired (Advanced Geno Techs Co.), and this was used as a BAC expression vector for the gene cluster of the human Fcγ receptor family (Fig. 1).

### Example 2: Selection of BACs comprising genomic sequences of human CD20 and mouse CD20 genes

Based on the cDNA sequence of the mouse CD20 gene (GenBank: BC028322), a mouse genomic DNA sequence was searched using UCSC Genome Browser. As a result, it was found that the mouse CD20 gene is present in the 198.19 cM region of chromosome 19. The cDNA sequence of the mouse CD20 gene was aligned together with the mouse genomic sequence, and as a result, it was revealed that the coding region of the mouse CD20 gene also encodes the 15-kb genomic sequence comprising 7 exons.

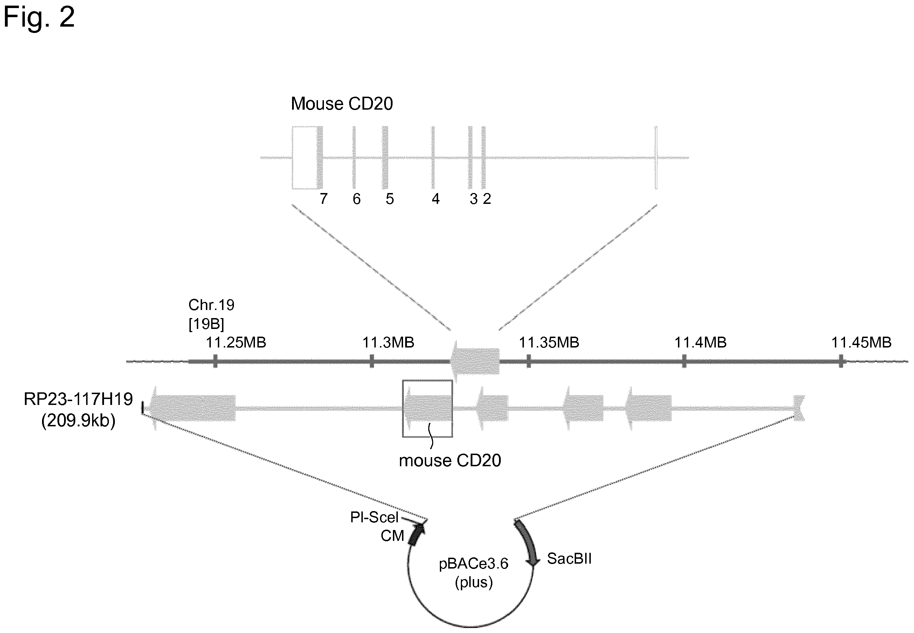

Subsequently, a BAC clone encoding the mouse CD20 gene locus was screened for, and as a result, RP23-117H19 (Fig. 2) that is a BAC clone, which covers 209.9 kb of chromosome 19 comprising a 111-kb genomic sequence at the 5'-upstream and a 83-kb genomic sequence at the 3'-downstream, as well as the genomic DNA sequence of the mouse CD20 gene locus, was identified and acquired (Advanced Geno Techs Co.). From the aforementioned gene information, it can be assumed that this 209.9-kb BAC clone, RP23-117H19, comprises all of expression control sequences that drive the CD20 gene. We determined that it would be rational to construct an expression vector for the human CD20 gene by utilizing this clone.

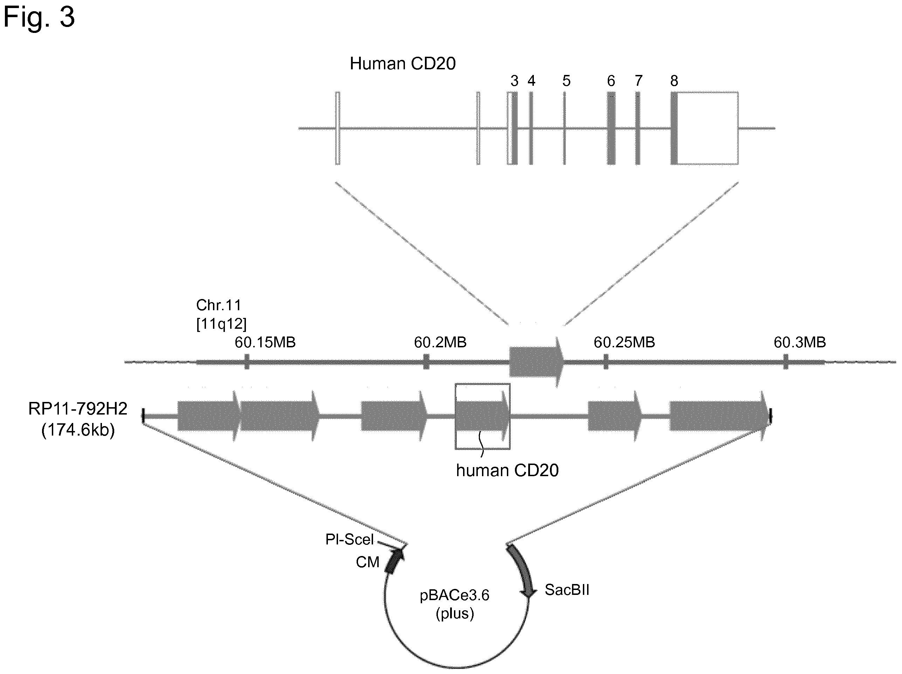

On the other hand, based on the cDNA sequence of the human CD20 gene (GenBank: BC002807), a human genomic DNA sequence was searched using UCSC Genome Browser. As a result, it was found that the human CD20 gene is present in the 11q12 region of chromosome 11. The cDNA sequence of the human CD20 gene was aligned together with the human genomic sequence, and as a result, it was revealed that the coding region of the human CD20 gene encodes the 14-kb genomic sequence comprising 8 exons. Subsequently, a BAC clone encoding a human CD20 gene locus was screened for, and as a result, RP11-792H2 (Fig. 3) that is a BAC clone with a size of 174.6 kb, comprising all of the genomic DNA sequences of the human CD20 gene locus, was identified and acquired (Advanced Geno Techs Co.).

### Example 3: Construction of recombinant BAC expression vector for human CD20 gene

Upon construction of a recombinant BAC expression vector for the human CD20 gene, the sequence of intron 4 of the human CD20 gene, the sequences of both termini of the human CD20 gene genomic DNA sequence of from the translation start codon of the human CD20 gene to the translation start codon of the same, and the 3'-terminus and 5'-terminus of the mouse CD20 gene genomic DNA sequence of from the translation start codon of the mouse BAC clone CD20 gene to the stop codon of the same, as shown below, were used as key sequences for an active homologous recombination reaction in *Escherichia coli.*
CD20-H1: 5'- ATGACAACAC CCAGAAATTC AGTAAATGGG ACTTTCCCGG CAGAGCCAAT-3' (SEQ ID NO: 1)
CD20-H2: 5'-CTCCCCAAGATCAGGAATCCTCACCAATAGAAAATGACAGCTCTCCTTAA-3' (SEQ ID NO: 2)
CD20-H3: 5'-CCTAGACATTTAGACTAGATAGCAAGATGTTTTGGAAAGCAAGAGGCAGC-3' (SEQ ID NO: 3)
CD20-H4: 5'-AGGAACATCCACTTCCATCTACCCCTTCTTGCTTACAATTCTGTTTGGTT-3' (SEQ ID NO: 4)
CD20-H5: 5'-AAGACTCTGATCTCACCTCACTGTCTTATTTTCAGGCGTTTGAAAACTCA-3' (SEQ ID NO: 5)
CD20-H6: 5'-ACTCTTTTCTTTTCTAAGCATTATTGTTTAGAGAGCTTCCAAGACACATA-3' (SEQ ID NO: 6)

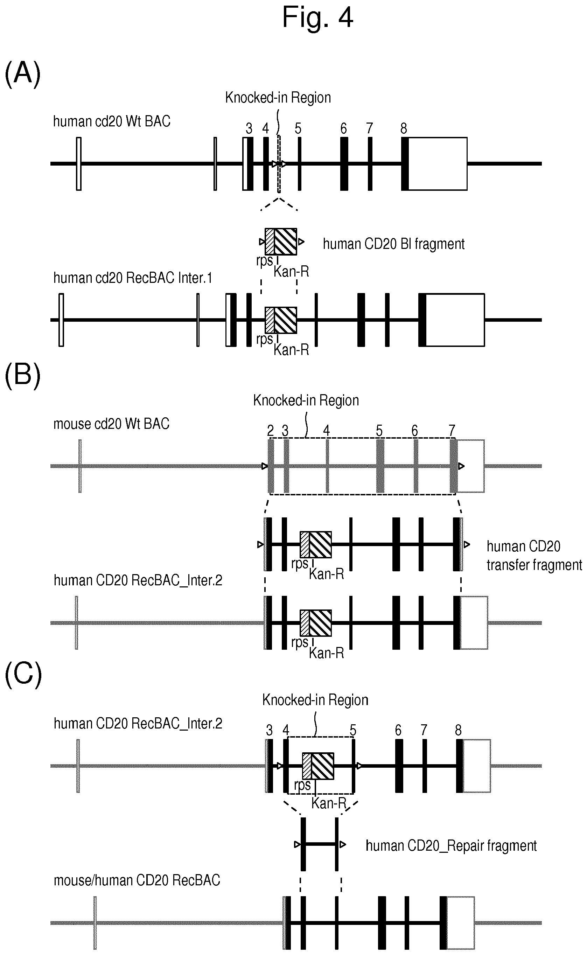

A schematic view of a method of constructing a recombinant BAC expression vector for the human CD20 gene is shown in Fig. 4. Hereafter, the construction of a recombinant BAC clone will be described based on individual views in Fig. 4.

Fig. 4(A): In order to insert a positive/negative selection marker cassette into the intron 4 of the human CD20 gene of the human BAC clone (human cd20 Wt BAC), a DNA fragment, in which CD20-H3 sequence, Rpsl-kan, and CD20-H4 sequence were ligated in tandem, was constructed by PCR using LA-Taq (Takara Bio, Inc.) (human CD20 BI fragment). A human BAC clone (human cd 20 Wt BAC) comprising the human CD20 gene locus and the human CD20 BI fragment were introduced into an *Escherichia coli* strain having the ability of a Red system, and the Red system was induced in this host *Escherichia coli.* Colonies resistant to chloramphenicol and kanamycin were picked up, so as to screen for a recombinant BAC clone, in which the human CD20 BI fragment was inserted into the intron 4 of the human CD20 gene of the CD20 gene locus (human CD20 RecBAC Inter. 1).

Fig. 4(B): Subsequently, in order to retrieve the genomic DNA sequence of an Rpsl-kan cassette-inserted human CD20 gene from the obtained human CD20 RecBAC Inter. 1 into a plasmid vector, a DNA cassette, in which CD20-H2-CD20-H6-SacB-pBluescript-CD20-H5-CD20-H1 were ligated in tandem, was constructed (CD20 pretransfer plasmid). By the same method as described above, a linearized CD20 pretransfer plasmid, as well as the human CD20 RecBAC Inter. 1, was introduced into an *Escherichia coli* strain having the ability of a Red system, and thereafter, the Red system was induced in this host *Escherichia coli.* Colonies resistant to ampicillin and kanamycin were picked up, so as to screen for a plasmid having the genomic DNA sequence of the Rpsl-kan cassette-inserted human CD20 gene (human CD20 transfer plasmid). Subsequently, by the restriction enzyme reaction, the genomic DNA sequence of an Rpsl-kan cassette-inserted human CD20 gene having, at both the termini, a CD20-H5 sequence and a CD20-H6 sequence derived from the mouse genomic sequence was cut out from the human CD20 transfer plasmid (i.e., human CD20 transfer fragment). By the same method as described above, the human CD20 transfer fragment, as well as a BAC clone (mouse cd20 Wt BAC) encoding mouse CD20, was introduced into an *Escherichia coli* strain having the ability of a Red system, and thereafter, the Red system was induced in this host *Escherichia coli.* Colonies resistant to chloramphenicol and kanamycin were picked up, so as to screen for a recombinant BAC clone of the mouse CD20/human CD20 gene, in which the mouse CD20 gene genomic DNA sequence of from the translation start codon of the mouse CD20 gene to the stop codon of the same was exactly substituted with the human CD20 gene genomic DNA sequence of from the translation start codon of the human CD20 transfer fragment to the stop codon of the same (i.e., human CD20 RecBAC Inter. 2).

Fig. 4(C): Finally, the intron 4 sequence of the human CD20 gene of the human CD20 gene locus was amplified by PCR, followed by negative selection, to obtain a DNA sequence used to remove Rpsl-kan that had been inserted into the intron 4 of the human CD20 gene (human CD20 Repair fragment). By the same method as described above, the human CD20 Repair fragment, as well as the human CD20 RecBAC Inter.2, was introduced into an *Escherichia coli* strain having the ability of a Red system, and thereafter, the Red system was induced in this host *Escherichia coli.* Colonies resistant to chloramphenicol and streptomycin were picked up, in order to construct a recombinant BAC clone of the mouse CD20/human CD20 gene, in which the mouse CD20 gene genomic DNA sequence of from the translation start codon of the mouse CD20 gene to the stop codon of the same was exactly substituted with the human CD20 gene genomic DNA sequence of from the translation start codon of the human CD20 gene to the stop codon of the same (i.e., mouse/human CD20 RecBAC). By sequence analysis, the genomic DNA sequence connected by the Red system was confirmed.

The obtained BAC clone was used as a recombinant BAC expression vector for human CD20 gene.

### Example 4: Purification of BAC expression vector for gene cluster of human Fcγ receptor family and recombinant BAC expression vector for human CD20 gene

*Escherichia coli* (DH10B strain) cells, which had been transformed with the BAC expression vector for the gene cluster of the human Fcγ receptor family and with the human CD20 recombinant BAC expression vector, respectively, were each cloned on an LB agar medium containing chloramphenicol. Thereafter, a single colony was picked up, and was then subjected to shaking culture in a liquid medium whole day and night. According to a partially modified method of Abe et al. (Exp Anim. 200453(4): 311-20. Establishment of an efficient BAC transgenes is protocol and its application to functional characterization of the mouse Brachyury locus. Abe K, Hazama M, Katoh H, Yamamura K, Suzuki M.), the BAC expression vector for the gene cluster of the human Fcγ receptor family and the human CD20 recombinant BAC expression vector were each purified using a plasmid extraction kit (MACHEREY-NAGEL, Nucleobond BAC 100 Kit). Thereafter, PI-SceI was added to the thus purified vectors, which were then reacted at 37°C for 16 hours for linearization. Subsequently, the linearized BAC expression vector for the gene cluster of the human Fcγ receptor family and the linearized human CD20 recombinant BAC expression vector were each applied to 1% SeaKem GTG agarose gel (Takara Bio, Inc.), and were then electrophoresed using a pulsed filed electrophoresis apparatus (BioRad, CHEFDR-II) under conditions of 6 v/cm, 0.1-40 sec, 15 hr, and 14°C. A part of the sample was visualized by a UV transilluminator used as a guide marker, so that the linearized BAC expression vector for the gene cluster of the human Fcγ receptor family and the linearized human CD20 recombinant BAC expression vector, which were resolved (or separated) in the agarose gel, were cut out using a razor, without performing UV irradiation. The obtained long-chain DNA fragments were extracted from the agarose gel by electric elution, and were then dialyzed against a TE buffer prepared for microinjection at 4°C for 2 hours. The purified DNA fragments were applied to pulsed-field electrophoresis, and it was confirmed that the long-chain DNA fragments were highly purified without being cleaved. Using NanoDrop spectrophotometer (Asahi Techno Glass Co., Ltd.), their DNA concentrations were determined. A solution of each DNA fragment was diluted to 0.5 ng/µl, and the solution of the BAC expression vector for the gene cluster of the human Fcγ receptor family and the solution of the recombinant BAC expression vector for the human CD20 gene, which were to be used to produce transgenic mice, were prepared.

### Example 5: Introduction of BAC expression vector for gene cluster of human Fcγ receptor family and recombinant BAC expression vector for human CD20 gene into fertilized eggs, and transplantation of gene-introduced fertilized eggs into surrogate animals.

5 IU pregnant mare's serum gonadotropin (PMSG: ASKA Pharmaceutical Co., Ltd.) was intraperitoneally injected into female Balb/c mice (Charles River Japan) and female C57BL/6JJcl mice (CLEA Japan, Inc.), and 48 hours after the injection, 5 IU human chorionic gonadotropin (hCG: ASKA Pharmaceutical Co., Ltd.) was intraperitoneally injected into each mouse, so as to induce superovulation. These female mice were crossed with male mice of the same lineage, and pronuclear embryos were then collected. As a result of the induction of superovulation by administration of PMSG and hCG and the subsequent crossing, 2029 pronuclear stage embryos were collected from Balb/c female mice, and 642 pronuclear stage embryos were collected from C57BL/6JJcl female mice.

The solution of the purified BAC expression vector for the gene cluster of the human Fcγ receptor family was mixed with an equal amount of the solution of the purified recombinant BAC expression vector for the human CD20 gene, and thereafter, the mixed solution was directly injected into the male nucleus of the pronuclear stage embryos of the Balb/c mice and the C57BL/6JJcl mice. The microinjected mouse fertilized eggs were observed under microscope. As a result, it was found that only a small number of fertilized eggs were sterile due to damage caused by injection operations, and that 1682 and 603 fertilized eggs maintained a fertile condition even after they had been subjected to microinjection. These fertilized eggs, into each of which the expression vector could be injected without damage, could be then transplanted into pseudopregnant mice.

The fertilized eggs of the Balb/c mice and the C57BL/6JJcl mice, into which the BAC expression vector for the gene cluster of the human Fcγ receptor family and the recombinant BAC expression vector for the human CD20 gene had been injected, were transplanted into surrogate mice, and after the pregnancy period of approximately 3 weeks, 104 and 109 offsprings were obtained from the Balb/c mice and the C57BL/6JJcl mice, respectively. Founder candidate individuals of the transgenic mice expressing the human Fcγ receptor family and the human CD20 (hereinafter referred to as "CD20 human antigen mice") were weaned when they were 4 weeks old, and ear tags were added to them for individual identification. Thereafter, tail tissues of the mice were obtained by biopsy, and were then preserved at -80°C until the tissues were subjected to analysis.

### Example 6: Selection of CD20 human antigen mice

The tail tissues from the founder candidates of the CD20 human antigen mice, which had been preserved at -80°C, were thawed at room temperature, and CD20 human antigen mice were then selected according to the following Southern blot hybridization method. Specifically, a lysis buffer comprising 1% SDS (Wako Pure Chemical Industries, Ltd.), 1 mg/ml Actinase E (Kaken Pharmaceutical Co., Ltd.) and 0.15 mg/ml Protenase K (Merck) was added to the tail tissues and s then shaken at 55°C for 16 hours, so that the tissues were solubilized. The tissues were subjected to phenol extraction and phenol/chloroform extraction, so that proteins binding to solubilized genomic DNA were removed from the tissues. RNA contained in the genomic DNA was decomposed by RNase A (Sigma), and high-molecular-weight genomic DNA was then precipitated by isopropanol precipitation. The precipitated genomic DNA was washed with 70% ethanol and was then air-dried, and it was then dissolved again in 50 µl TE. The DNA concentration of the genomic DNA solution prepared from each specimen was determined by the absorption method, and the volume of a genomic DNA solution corresponding to 5 µg DNA was then calculated from the value of the DNA concentration of each specimen. The BAC expression vectors used in microinjection were diluted to 1, 3, 10, and 30 copies, and 5 µg of the genomic DNA of control mice, which had been prepared separately, was then added thereto, so as to prepare positive control DNA for Southern blot analysis. On the other hand, 5 µg of the genomic DNA of control mice was used as negative control DNA for Southern blot analysis. The restriction enzyme XbaI or HincII was added to the genomic DNA, the positive control DNA, and the negative control DNA, which had been prepared from individual specimens, and thereafter, the restriction enzyme reaction was carried out at 37°C for 16 hours. The generated genomic DNA fragment was precipitated by isopropanol precipitation, was then washed with 70% ethanol, and was then air-dried. Thereafter, it was dissolved again in TE. The XbaI or HincII fragments of those genomic DNA were applied to 1.2% agarose gels, and were then electrophoresed. Then, the genomic DNA fragments resolved (or separated) in the agarose gel were visualized by using a UV transilluminator, and were then photographed with scale. Each agarose gel was immersed in 0.25 N hydrochloric acid, and was then shaken gently for 10 minutes. Thereafter, the agarose gel was immersed in 0.4 N sodium hydroxide, and was further shaken gently for 10 minutes. The XbaI or HincII fragments of the genomic DNA separated in the agarose gel was transferred onto a nylon membrane (Hybond-XL; GEH) at room temperature for 16 hours according to the capillary method using 0.4 N sodium hydroxide. The nylon membrane, onto which the XbaI or HincII fragments of the genomic DNA had been transferred, was immersed in 2 x SSC and was gently shaken for 10 minutes. Thereafter, the membrane was air-dried, and was then preserved at room temperature until it was used in hybridization. Using a DNA labelling kit (Megaprime DNA Labelling System; GEH), a human CD20 probe fragment cloned from the human CD20 recombinant BAC expression vector, and an FCγRIIIa probe fragment cloned from the BAC expression vector for the gene cluster of the human Fcγ receptor family were each labeled with [³²P] according to the random prime method. Using a Sephadex spin column (ProbeQuant G-50 Micro Columns; GEH), the thus [³²P]-labeled fragments were purified to prepare a [³²P]-labeled human CD20 probe and a [³²P]-labeled human FcγRIIIa probe, respectively. The nylon membrane, onto which the XbaI or HincII fragments of the genomic DNA had been transferred, was put in a hybridization buffer, and it was then preincubated at 65°C for 1 hour. Thereafter, the [³²P]-labeled human CD20 probe and the [³²P]-labeled human FcγRIIIa probe were heated at 95°C for 5 minutes, and immediately after the heating, they were cooled on ice for 5 minutes. The thus denatured probes were each added to the nylon membrane, onto which the XbaI or HincII fragments had been transferred, and they were then incubated at 65°C for 4 hours. After completion of the incubation, the nylon membrane was removed, and was then washed using 0.1% SDS and 0.5 x SSC at 65°C for approximately 15 minutes. Radioactivity derived from the probe binding to the membrane was monitored using a survey meter, and this washing operation was repeatedly carried out until such radioactivity became almost constant. The washed membrane was covered with Saran Wrap (registered trademark), an X-ray film (BioMax MS; Kodak) was then placed on the membrane in a dark room, and it was then placed in an autoradiography cassette. After exposure at 4°C for 1 week, the X-ray film was developed. Specific signals derived from the recombinant BAC expression vector for the human CD20 gene and the BAC expression vector for the gene cluster of the human Fcγ receptor family were detected by autoradiography.

As a result, specific signals derived from the human CD20 recombinant BAC expression vector and the BAC expression vector for the gene cluster of the human Fcγ receptor family, which were all positive controls, could be detected from the fragments of the aforementioned vectors as the results of the hybridization with the [³²P]-labeled human CD20 probe and the [³²P]-labeled human FcγRIIIa probe. Moreover, the expression vector-derived hybridization signals could be detected from all of the positive controls. Accordingly, it was found that one or more copies of expression vectors, which have been integrated into the host genome, can be detected by Southern analysis using the [³²P]-labeled probes.

Furthermore, as a result of hybridization of a genomic DNA fragment extracted from offsprings that can be founder candidates of the CD20 human antigen mice, with a [³²P]-labeled probes, the individuals, which give specific signals by double hybridization with the [³²P]-labeled human CD20 probe and with the [³²P]-labeled human FcγRIIIa probe, were identified to be CD20 human antigen mice, namely, as founder individuals. As a result, a total of 15 founders of CD20 human antigen mice, into which both the human CD20 recombinant BAC expression vector and the BAC expression vector for the gene cluster of the human Fcγ receptor family had been introduced, could be identified.

The signal strength of each specimen was compared with the signal strength of positive control DNA, and the copy number of the expression vectors introduced into the genome was estimated. As a result, the copy number of the expression vectors introduced into these founders of CD20 human antigen mice was found to be 1 to 30 copies. Apart from the founders of CD20 human antigen mice, founders of transgenic mice into which only the human CD20 recombinant BAC expression vector had been introduced, and founders of transgenic mice into which only the BAC expression vector for the gene cluster of the human Fcγ receptor had been introduced, were each obtained.

### Example 7: Establishment of lineages from founders of CD20 human antigen mice

Male founders of CD20 human antigen mice, into which 3 or more copies of the human CD20 recombinant BAC expression vector and the BAC expression vector for the gene cluster of the human Fcγ receptor family had been introduced, were allowed to grow up to a crossing season, and thereafter, their lineages were established according to the following *in vitro* fertilization method. Specifically, the epididymis was excised from each male mouse, and thereafter, the sperm to be used in the *in vitro* fertilization was collected from the tail portions thereof, and was then pre-cultured in a TYH solution for 2 hours. 5 IU PMSG was intraperitoneally injected into 3- to 4-week-old female Balb/c mice and C57BL/6JJcl mice, and 48 hours after the injection, 5 IU hCG was intraperitoneally injected into each mouse, so that superovulation was induced. The thus superovulation-induced female Balb/c mice and C57BL/6JJcl mice were euthanized by cervical dislocation 16 to 18 hours after the administration of hCG. The oviduct was excised from each mouse, and unfertilized eggs were recovered from the ampulla of the oviduct, and were then added to an HTF solution. A portion of the pre-cultured sperm derived from the founders of CD20 human antigen mice was added to the HTF solutions comprising the unfertilized eggs and then incubated for 4 to 6 hours. Thereafter, it was transferred into an mWM solution, and was further cultured *in vitro* for 24 hours to obtain fertilized eggs as offsprings of F1 individuals. From among the fertilized eggs obtained as a result of the *in vitro* fertilization, only embryos that had reached the two-cell stage were recovered, and approximately 20 embryos were transplanted into the oviducts of each pseudopregnancy-induced recipient female mouse, so as to establish lineages from the founders of CD20 human antigen mice. Offsprings obtained as a result of natural delivery were bred until weaning. These F1 individuals of the CD20 human antigen mice were weaned when they were 4 weeks old, and ear tags were then attached to them for individual identification. Thereafter, tail tissues were obtained by biopsy, and were then preserved at -80°C until the tissues were subjected to analysis.

The tail tissues from the F1 individuals of the CD20 human antigen mice, which had been preserved at -80°C, were melted at room temperature, and the male F1 individuals of the CD20 human antigen mice were identified by the same Southern blot hybridization method as described above, in which the [³²P]-labeled human CD20 probe and the [³²P]-labeled human FcγRIIIa probe were used.

As a result, from four out of the founders of the CD20 human antigen mice subjected to the establishment of a lineage, a plurality of F1 mice, to which transgenes were transmitted, could be obtained. Considering the efficiency of establishing a lineage from these founder mice, it has been demonstrated that the expression vectors do not have a serious influence on the onset or development of embryos. Not only in the case of the founders of the CD20 human antigen mice, but also in the case of two founders of transgenic mice into which only the human CD20 recombinant BAC expression vector had been introduced and one founder of transgenic mice into which only the BAC expression vector for the gene cluster of the human Fcγ receptor family had been introduced, a plurality of F1 individuals, into which the transgenes were transmitted from the founders, could be obtained.

### Example 8: Analysis of expression of human antigen in CD20 human antigen mice

The F1 individuals of the CD20 human antigen mice, the lineages of which had been successfully established, were euthanized by cervical dislocation. The whole body was cleaned with ethanol, and the abdominal portion was then cut with dissecting scissors to remove the spleen therefrom. The removed spleen was placed in a plastic petri dish, to which a medium had been added and which had been then cooled on ice. The spleen was loosen to cell suspension with a pair of tweezers, and was then subjected to pipetting and filtration. Thereafter, the spleen cells were resuspended in a Tris-buffered ammonium chloride solution, so as to remove erythrocytes. The number of living cells in the prepared splenic cell suspension was counted. In order to block the non-specific binding of mouse CD16 and CD32 present in the splenic cells with fluorescently labeled IgG, an anti-mouse CD16/CD32 antibody (Nippon Becton Dickinson Company, Ltd.) was added to an Eppendorf tube containing the suspension of 1x10⁶ splenic cells, and was then incubated at 4°C for 30 minutes. Thereafter, the supernatant was removed by centrifugation, followed by resuspension. After that, a PE-labeled antibody and an FITC-labeled antibody (Nippon Becton Dickinson Company, Ltd.) were added to the suspension, which was then incubated at 4°C for 30 minutes. After centrifugation followed by resuspension for washing, a 7-AAD nuclear staining solution was added and the mixture was incubated at room temperature in a dark place for 10 minutes, so that dead cells were stained. The thus stained cell suspension was analyzed using a flow cytometer, and dot plots were output by multiple staining, so that the expression of the human antigen in the CD20 human antigen mice was analyzed.

As a result, it was found that, in the negative control (wild-type) mice, there were no found cells, which should be double positive in staining with the FITC-anti-mouse B220 antibody and with the PE-anti-human CD20 antibody, whereas all of four successfully established lineages from the CD20 human antigen mice had, at a high rate, human CD20-expressing B cells, which were double positive in staining with the FITC-anti-mouse B220 antibody and with the PE-anti-human CD20 antibody. In addition, it was also found that two out of three successfully established lineages from the transgenic mice, into which only the human CD20 recombinant BAC expression vector had been introduced, had, at a high rate, human CD20-expressing B cells, which were double positive in staining with the FITC-anti-mouse B220 antibody and with the PE-anti-human CD20 antibody.

Moreover, it was also found that two out of four successfully established lineages from the CD20 human antigen mice had human CD20-expressing B cells at a high rate, and also had human FcγRIIIa-expressing NK cells (Fig. 5 and Fig. 6). These 2 lineages (a Balb/c-9_6 lineage and a C57BL/6-1_2 lineage) were established as CD20 human antigen mice.

According to histogram analysis, the percentages of human CD20-expressing B cells and human FcγRIIIa-expressing NK cells in the B cell fraction, macrophage cell fraction and NK cell fraction in the splenic cells of the Balb/c-9_6 lineage were examined. As a result, the percentages of the cells of each type were confirmed to be 44%, 65% and 21%, respectively. These results showed that the human CD20 antigen and the human FcγRIIIa antigen are tissue-specifically expressed in the Balb/c-9_6 lineage, and thus, the Balb/c-9_6 lineage was used in the subsequent bioassays.

### Example 9: In vivo assay of therapeutic anti-CD20 antibody drug utilizing CD20 human antigen mice

The CD20 Human antigen mice, the Balb/c-9_6 lineage mice, and the male F1 individuals were bred until 12 weeks old, and thereafter, fertilized eggs of F2 generation were obtained as a result of the *in vitro* fertilization of the CD20 human antigen mice and the male F1 individuals with female wild-type Balb/c lineage mice by the same method as that described above. From among these fertilized eggs, only embryos that had reached the two-cell stage were recovered, and approximately 400 fertilized eggs were transplanted into the oviducts of pseudopregnancy-induced recipient female mice, as many as approximately 20 eggs per mouse. F2 individuals of the CD20 human antigen mice, which were born by natural delivery, were weaned when they were 4 weeks old. According to the above-described Southern blot hybridization method using the [³²P]-labeled human CD20 probe and the [³²P]-labeled human FcγRIIIa probe, F2 individuals of the CD20 human antigen mice were selected. A therapeutic anti-CD20 antibody drug was administered to these F2 individuals of the CD20 human antigen mice, and the individuals were compared in terms of an effect (or an action) to remove splenic B lymphocytes.

### (1) Confirmation of dose dependency of splenic B lymphocyte depletion effect by administration of anti-CD20 antibody drug

The F2 individuals of CD20 human antigen mice were assigned to individual experiment groups, and BM-ca, Rituximab, Ofatumumab, and negative control IgG (Infliximab) were then administered to each group. Thereafter, the dose dependency of the splenic B lymphocyte depletion effect was examined. Specifically, 50 µg and 250 µg of BM-ca, Rituximab, and Ofatumumab were intraperitoneally administered to the F2 individuals of CD20 human antigen mice in each experiment group by a single administration. The day at which the antibody was administered was defined as Day 0. On Day 7, the CD20 human antigen mice in each experiment group were euthanized by cervical dislocation, and the spleen was then excised from each mouse. The splenic cells immunostained with the FITC-anti-mouse B220 antibody and the PE-anti-human CD20 antibody were analyzed using a flow cytometer by the same method as that described above, and based on the obtained dot plots, the number of splenic B lymphocytes in the CD20 human antigen mice, to which the therapeutic anti-CD20 antibody drug had been administered, was evaluated.

As a result, in the group of 250-µg Infliximab administration, no change was found in the number of splenic B lymphocytes, whereas in the group of 250-µg BM-ca, Rituximab, and Ofatumumab administration, the number of splenic B lymphocytes remarkably decreased.

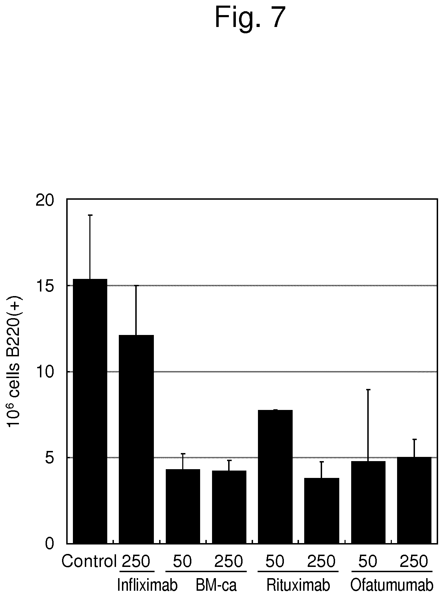

When 50 µg and 250 µg Rituximab administration groups were compared with each other, the number of splenic B lymphocytes decreased depending on the dose of the administered Rituximab. Moreover, in the BM-ca administration group and the Ofatumumab administration group, wherein BM-ca and Ofatumumab are second generation antibodies having stronger biological activity than Rituximab, the number of splenic B lymphocytes decreased to the maximum only by administration of a low dose of antibody (50 µg) (Fig. 7).

### (2) Confirmation of change with time in the effect to remove B cells by administration of anti-CD20 antibody drug

F2 individuals of the CD20 human antigen mice were assigned to individual experiment groups, and BM-ca, Rituximab, Ofatumumab, and Infliximab were then administered to each group. Thereafter, the change with time in the effect to remove splenic B lymphocytes was examined. Specifically, 250 µg of BM-ca, Rituximab, Ofatumumab and Infliximab were intraperitoneally administered to the F2 individuals of the CD20 human antigen mice in each group by a single administration. The day at which the antibody was administered was defined as Day 0. On Day 2, Day 7, Day 14 and Day 21, the CD20 human antigen mice in each experiment group were euthanized by cervical dislocation, and the spleen was then excised from each mouse. The splenic cells were immunostained with the FITC-anti-mouse B220 antibody and the PE-anti-human CD20 antibody by the same method as that described above, and were then analyzed using a flow cytometer. Based on the obtained dot plots, the number of B cells in the CD20 human antigen mice, to which the therapeutic anti-CD20 antibody drug had been administered, was evaluated.

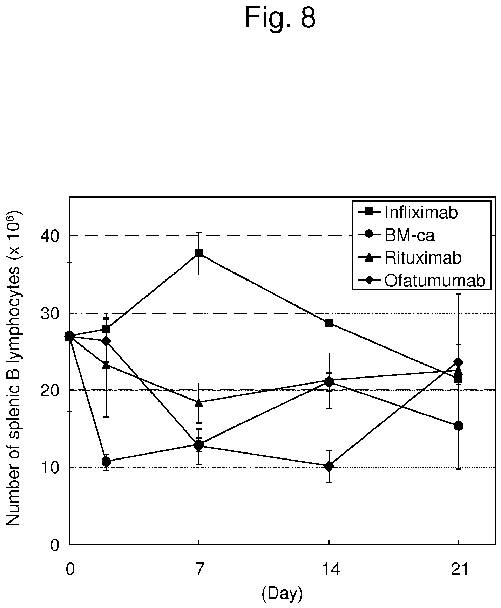

As a result, in the Infliximab administration group, a remarkable decrease was not found in the number of splenic B lymphocytes. In the Rituximab and Ofatumumab administration groups, so remarkable change was not found on Day 2, but on Day 7 and Day 14, the number of splenic B lymphocytes was remarkably decreased, and on Day 21, the number of the cells was almost recovered. On the other hand, in the BM-ca administration group, a decrease in the number of splenic B lymphocytes has already reached to almost the maximum on Day 2, and the effect was sustained until Day 7 and then, were gradually recovered (Fig. 8).

From the aforementioned results, it has been demonstrated that the *in vivo* effects of a therapeutic anti-CD20 antibody drug can be evaluated as pharmacodynamic data that exactly reflect the administration dose and time course, according to the *in vivo* assay using the CD20 human antigen mice. Moreover, based on the *in vitro* experiment using tumor cells, it has been reported that BM-ca has strong direct cytotoxicity, which Rituximab and Ofatumumab do not have (Kobayashi, H. et al., CANCER MEDICINE, 2: 130-143 (2013)). Further, it has been elucidated that, in the clinical phase I trial, in which recurrent low-grade B-cell non-Hodgkin's lymphoma patients are used as subjects, BM-ca provides the same antitumor effect as that of Rituximab even in an administration period that is a half of the administration period of Rituximab that is a current standard treatment (Tobinai, K. et al., Journal of Clinical Oncology, 31, 2013 (suppl; abstr 8551)). The lowest dose of BM-ca that exhibited the antitumor effect in the clinical phase I trial is 10 mg/kg, and this dose corresponds to the dose in which 250 µg of BM-ca is administered to a mouse. In this *in vivo* assay using such CD20 human antigen mice, BM-ca exhibited a B cell depletion effect in an extremely short time or at a low dose, when compared with Rituximab and Ofatumumab. By evaluating these results in combination with the aforementioned findings, it has been demonstrated that pharmacological effects reflecting the biological characteristics of an anti-CD20 antibody drug can be observed by performing the *in vivo* assay using the CD20 human antigen mice, and thus that extremely high-quality information can be obtained to estimate the dose applied in a clinical trial.

### Example 10: Effect of human Fcγ receptor family in in vivo assay using CD20 human antigen mice

BM-ca or Rituximab was administered to each of CD20 human antigen mice and transgenic mice, into which only the human CD20 recombinant BAC expression vector had been introduced, and then, the effect of human-type effector cells expressing the human Fcγ receptor family on the effect to remove splenic B lymphocytes by administration of the anti-CD20 antibody drug were examined.

To F2 individuals of the CD20 human antigen mice (Balb/c-9_6 lineage), and to F1 individuals of transgenic mice (Balb/c-10_8 lineage) into which only the human CD20 recombinant BAC expression vector had been introduced, 250 µg of BM-ca and Rituximab were each intraperitoneally administered by a single administration. The day at which the antibody was administered was defined as Day 0. On Day 7, the mice in each experiment group were euthanized by cervical dislocation, and the spleen was then excised from each mouse. The splenic cells were immunostained with the FITC-anti-mouse B220 antibody and the PE-anti-human CD20 antibody by the same method as that described above, and were then analyzed using a flow cytometer. Based on the obtained dot plots, the number of splenic B lymphocytes in the mice of each experiment group, to which the therapeutic anti-CD20 antibody drug had been administered, was evaluated.

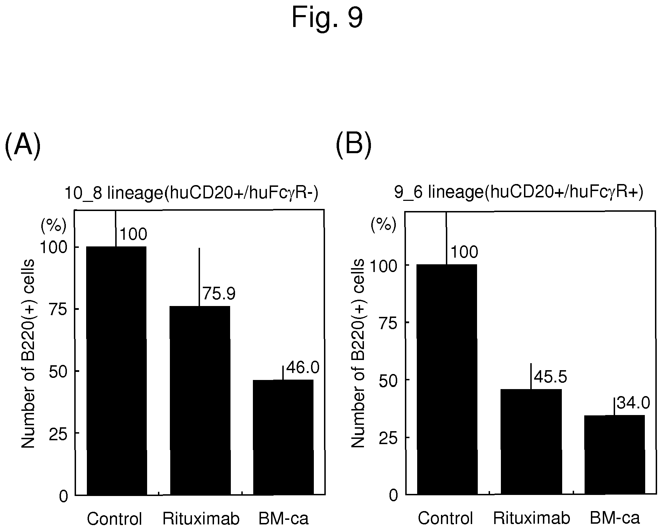

As a result, in a group in which Rituximab was administered to the CD20 human antigen mice, the average number of splenic B lymphocytes was reduced to 45.5%, when compared with a control group (Fig. 9 (B)), and in the group in which Rituximab was administered to the transgenic mice into which only the human CD20 recombinant BAC expression vector had been introduced, the reduction in the average number of splenic B lymphocytes remained at 75.9%, when compared with the control group (Fig. 9 (A)).

From these results, it has been demonstrated that the effect to remove splenic B lymphocyte by ADCC activity, which is mediated by the activation of effector cells by Rituximab, can be significantly detected because of the presence of human-type effector cells expressing the human Fcγ receptor in the CD20 human antigen mice.

Even in a case where BM-ca was administered to the CD20 human antigen mice, the average number of splenic B lymphocytes in the administration group was reduced to 34.0%, when compared with a control group (Fig. 9 (B)). On the other hand, in a case where BM-ca was administered to the transgenic mice into which only the human CD20 recombinant BAC expression vector had been introduced, the reduction in the average number of splenic B lymphocytes in the administration group reached 46.0% (Fig. 9 (A)). From these results, it has been demonstrated that BM-ca has strong direct cytotoxicity and thus, has the effect to remove splenic B lymphocytes before inducing the activation of the effector cells in the CD20 human antigen mice.

### Example 11: Test for pharmacological effect of therapeutic anti-CD20 antibody drug on disease mouse model in which CD20 human antigen mice are utilized

CD20 human antigen mice, C57BL/6-1_2 lineage mice, and male Flindividuals were bred until their crossing season, and thereafter, the fertilized eggs of F2 generation were obtained as a result of the *in vitro* fertilization of the CD20 human antigen mice and the male F1 individuals with female wild-type C57BL/6 lineage mice by the same method as that described above. From among these fertilized eggs, only embryos that had reached the two-cell stage were recovered, and approximately 400 fertilized eggs were transplanted into the oviducts of the pseudopregnancy-induced recipient female mice, at a rate of approximately 20 eggs per mouse. F2 individuals of the CD20 human antigen mice, which were born by natural delivery, were weaned when they were 4 weeks old. According to the above-described Southern blot hybridization method using the [³²P]-labeled human CD20 probe and the [³²P]-labeled human FcγRIIIa probe, F2 individuals of the CD20 human antigen mice were selected.

Thereafter, CD20 human antigen mice obtained in the F2 individuals of the CD20 human antigen mice were successively crossed with genetically modified and primary biliary cirrhosis model mice (i.e., disease model mice), and the thus generated mice were weaned when they were 4 weeks old. According to PCR, CD20 human antigen-disease model mice were selected. From among these mice, a total of approximately 20 female individuals were selected, and were then subjected to a pharmacological effect test, in which the effect of an anti-CD20 antibody drug, BM-ca, to inhibit liver inflammation would be confirmed.

The selected female CD20 human antigen-disease model mice were assigned to individual experiment groups, and 250 µg of the therapeutic anti-CD20 antibody drug, BM-ca, and a solvent (PBS) were intraperitoneally administered to each mouse once a week over 16 weeks, repeatedly. During the administration of the therapeutic anti-CD20 antibody drug, blood was collected once 2 weeks, and the ratio of lymphocytes in peripheral blood immunostained with the FITC-anti-mouse CD19 antibody and the PE-anti mouse CD4 antibody was analyzed using a flow cytometer. Based on the obtained dot plots, the number of B lymphocytes in the peripheral blood of CD20 human antigen-disease model mice, to which the therapeutic anti-CD20 antibody drug had been administered, was evaluated.

Finally, the day at which the therapeutic anti-CD20 antibody drug and the solvent were administered was defined as Day 0. On Day 7, all of the CD20 human antigen-disease model mice were euthanized, and the liver was then excised from each mouse. A portion of the excised liver tissues was immobilized with a 10% neutral formalin solution to produce a paraffin block, and the section was then stained with hematoxylin-eosin. Thereafter, histopathological evaluation of liver inflammation was performed, and the inflammation scoring of the portal region and the parenchymal cell region was carried out. Moreover, a portion of the excised liver tissues was ground and suspended, and thereafter, the numbers of monocytes, CD8-positive T cells, and CD44 high expression CD8-positive T cells, which had infiltrated into the liver, were evaluated using a flow cytometer. As a control, only the solvent was administered in the same manner as described above, and the numbers of the cells of each type were then evaluated.

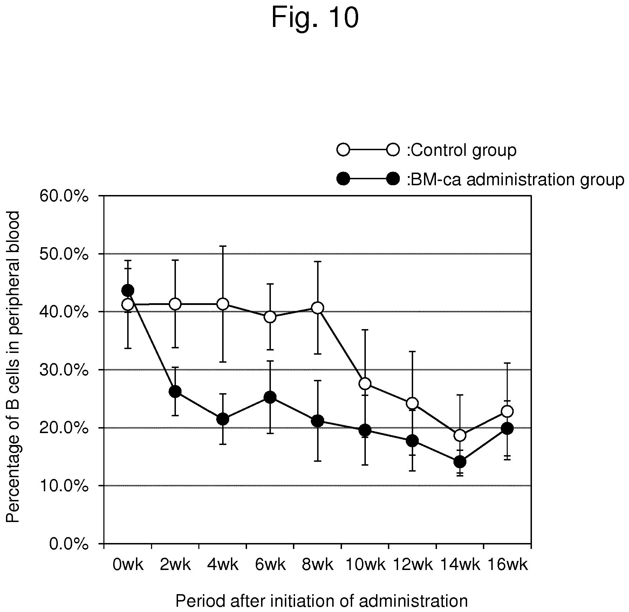

As a result, in the therapeutic anti-CD20 antibody drug administration group, the ratio of B lymphocytes in the peripheral blood of the CD20 human antigen-disease model mice was remarkably reduced immediately after administration of the drug, and the ratio was maintained at a low level over the entire administration period (Fig. 10).

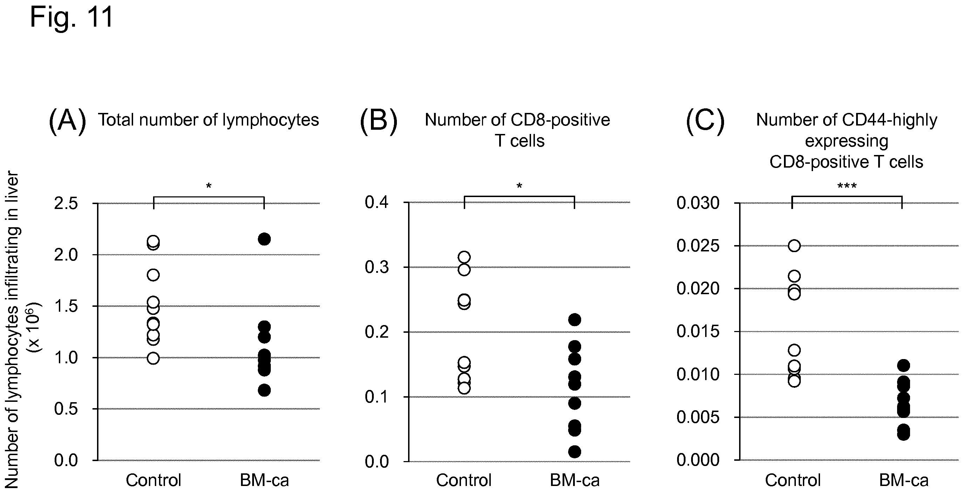

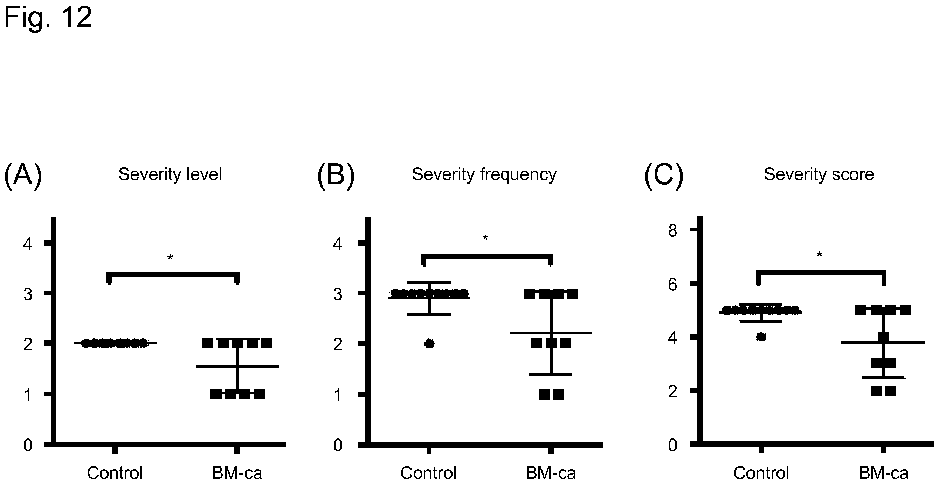

Moreover, the number of lymphocytes infiltrating into the livers of the CD20 human antigen-disease model mice was measured after administration of the therapeutic anti-CD20 antibody drug for 16 weeks. As a result, the total number of lymphocytes and the number of CD8-positive cytotoxic T cells, which had infiltrated into the livers of mice having primary biliary cirrhosis were significantly reduced by administration of the therapeutic anti-CD20 antibody drug, and in particular, the number of CD44-highly expressing and CD8-positive activated cytotoxic T cells was remarkably reduced (Fig. 11). Histopathological evaluation was carried out on liver tissues. As a result, reflecting the number of cytotoxic T cells infiltrating into the liver, the severity level and severity frequency of the substantial inflammatory area of the liver of the CD20 human antigen-disease model mice were each significantly improved by administration of the therapeutic anti-CD20 antibody drug (Fig. 12).

From the aforementioned results, it was confirmed that the CD20 human antigen mice according to the present invention can be utilized in pharmacological tests for confirming the pharmacological effect (or the efficacy), in which therapeutic anti-CD20 antibody drugs are administered to disease model mice and the therapeutic effects of the drugs on the mice are then confirmed. The CD20 human antigen mice according to the present invention is expected to promote the development of better antibody drugs having effects superior to the conventional antibody drugs, or introduction of the drugs into clinical trials, and also, the CD20 human antigen mice can be a useful tool that can also be used in the field of drug repositioning, in which the new application of previously clinically used antibody drugs is examined.

## Claims

1. A transgenic non-human animal, into which three or more copies of a human CD20 recombinant BAC expression vector and the BAC expression vector for genes encoding human Fcγ receptor family members have been introduced, and which expresses the genes encoding human CD20 and the genes encoding human Fcγ receptor family members, wherein:
(i) the human Fcγ receptor family members comprise human FcyRIIa, human FcyRIIb, human FcyRIIc, human FcyRIIIa, and human FcyRIIIb;
(ii) the genes encoding human CD20 are expressed through the mechanism that controls the expression of the homolog gene of the host non-human animal to the gene encoding human CD20; and
(iii) the transgenic non-human animal is selected from a mouse, rat, hamster, guinea pig, rabbit, dog, cat, bovine, sheep, horse, or swine.

2. A method for evaluating a property of a test substance, comprising: administering a test substance to the transgenic non-human animal according to claim 1; and then evaluating *in vivo* the property of the test substance in the transgenic non-human animal, wherein the test substance is an antibody which binds to human CD20 and has all or part of a peptide sequence of a human-derived gamma chain Fc region.

3. The method according to claim 2, wherein the property of the test substance is an ability to inhibit the function of human CD20, an ability to bind to human CD20, a cytotoxic activity to cells, tissues or organs expressing human CD20, biodistribution, pharmacological effects, or safety.

## Patentansprüche

1. Transgenes nichtmenschliches Tier, in das drei oder mehr Kopien eines rekombinanten Menschliches-CD20-BAC-Expressionsvektors und der BAC-Expressionsvektor für Gene, die Mitglieder der menschlichen Fcγ-Rezeptorfamilie eingeführt wurden und das die menschliches CD20 codierenden Gene und die Mitglieder der menschlichen Fcγ-Rezeptorfamilie codierenden Gene exprimiert, wobei:
(i) die Mitglieder der menschlichen Fcγ-Rezeptorfamilie menschliches FcγRIIa, menschliches FcγRIIb, menschliches FcγRIIc, menschliches FcγRIIIa und menschliches FcγRIIIb umfassen;
(ii) die menschliches CD20 codierenden Gene über den Mechanismus exprimiert werden, der die Expression des zum menschliches CD20 codierenden Gen homologen Gens des nichtmenschlichen Wirtstiers steuert; und
(iii) das transgene nichtmenschliche Tier aus Maus, Ratte, Hamster, Meerschweinchen, Kaninchen, Hund, Katze, Rind, Schaf, Pferd oder Schwein ausgewählt ist.

2. Verfahren zur Bewertung einer Eigenschaft einer Testsubstanz, umfassend: Verabreichen einer Testsubstanz an das transgene nichtmenschliche Tier nach Anspruch 1; und danach Bewerten der Eigenschaft der Testsubstanz beim transgenen nichtmenschlichen Tier in vivo, wobei es sich bei der Testsubstanz um einen Antikörper handelt, der an menschliches CD20 bindet und die gesamte oder einen Teil einer Peptidsequenz einer vom Menschen stammenden Gamma-Kette-Fc-Region aufweist.

3. Verfahren nach Anspruch 2, wobei es sich bei der Eigenschaft der Testsubstanz um eine Fähigkeit zur Hemmung der Funktion von menschlichem CD20, eine Fähigkeit zur Bindung an menschliches CD20, eine zytotoxische Aktivität gegenüber Zellen, Geweben oder Organen, die menschliches CD20 exprimieren, Bioverteilung, pharmakologische Wirkungen oder Sicherheit handelt.

## Revendications

1. Animal non humain transgénique, dans lequel trois copies ou plus d'un vecteur d'expression BAC recombinant de CD20 humain et du vecteur d'expression pour des gènes codant pour des membres de la famille de récepteurs Fcγ humains ont été introduits, et qui exprime les gènes codant pour CD20 humain et les gènes codant pour des membres de la famille de récepteurs Fcγ humains, dans lequel :
(i) les membres de la famille de récepteurs Fcγ humains comprennent FcγRIIa humain, FcγRIIb humain, FcγRIIc humain, FcγRIIIa humain et FcγRIIIb humain ;
(ii) les gènes codant pour CD20 humain sont exprimés par l'intermédiaire du mécanisme qui commande l'expression du gène de l'animal non humain hôte homologue au gène codant pour CD20 humain ; et
(iii) l'animal non humain transgénique est choisi parmi une souris, un rat, un hamster, un cobaye, un lapin, un chien, un chat, un bovin, un mouton, un cheval ou un porc.

2. Procédé d'évaluation d'une propriété d'une substance d'essai, comprenant : l'administration d'une substance d'essai à l'animal non humain transgénique selon la revendication 1 ; puis l'évaluation *in vivo* de la propriété de la substance d'essai chez l'animal non humain transgénique, dans lequel la substance d'essai est un anticorps qui se lie à CD20 humain et comporte tout ou partie d'une séquence peptidique d'une région Fc de chaîne gamma dérivée d'humain.

3. Procédé selon la revendication 2, dans lequel la propriété de la substance d'essai est une capacité à inhiber la fonction de CD20 humain, une capacité à se lier à CD20 humain, une activité cytotoxique sur des cellules, des tissus ou des organes exprimant CD20 humain, la biodistribution, les effets pharmacologiques ou la sécurité.
